# EUROPEAN PATENT SPECIFICATION

(11) **EP 2 570 150 B1**
(45) Date of publication and mention of the grant of the patent: **16.01.2019**
(21) Application number: 12275129.0
(22) Date of filing: 30.08.2012
(51) Int. Cl.: A61M 25/10, A61K 49/04, A61K 49/22

(54) **Ultrasonically visible balloon catheter assembly**
Durch Ultraschall sichtbare Scoring-Ballon-Anordnung
Ensemble de cathéter à ballonnet visible par ultrasons

(30) Priority: 16.09.2011 GB 201116075
(43) Date of publication of application: 20.03.2013
(73) Proprietor: Cook Medical Technologies LLC, Bloomington, IN 47404 (US)
(72) Inventor: Aggerholm, Steen, 4660 St. Heddinge (DK); Elgaard, Per, 4690 Haslev (DK)
(74) Representative: Georgiou, Sarah Caroline

(56) References cited:
- US-A- 4 364 392
- US-A- 5 147 631
- US-A1- 2002 054 852
- US-A1- 2009 123 385
- US-A1- 2010 010 470
- US-A1- 2010 305 389

## Description

### Technical Field

The present invention relates to an ultrasonically visible medical balloon assembly. The invention also encompasses balloon catheter assemblies which are echogenic and radiopaque.

### Background Art

The use of balloon catheters is well documented. Common uses include angioplasty procedures, vascular occlusion and for the deployment of implantable medical devices. Typically, such balloons are made of a thin but strong material, Pebax being one example, which is able to be wrapped into a very small diameter around the balloon catheter and yet which is strong when inflated. Cutting or scoring balloons typically have one or more cutting or scoring elements provided on or in the balloon wall which extend beyond the perimeter of the balloon wall in a radially outward direction.

As a result of the structure and materials used for such scoring balloons, they are not particularly suited for traditional angiographic imaging techniques, particularly ultrasonic imaging, thereby having to rely upon less advantageous methods such as fluoroscopy, X-ray or CRT imaging. Specifically, such balloons tend to be very difficult to see and in some instances can be virtually invisible to ultrasonic imaging. In order to avoid having to rely on any of these less advantageous imaging techniques, such balloons can be inflated with a contrast media which is opaque to ultrasonic waves. However, suitable contrast media does not resolve the imaging difficulties when the balloon is in its deflated condition. Moreover, contrast media tends to be relatively more viscous than other fluids which may be used to inflate such balloons, with the result that the time required for inflation and deflation of a balloon with contrast media is increased. The increased viscosity also limits the minimum diameter of the inflation and deflation channels which must be used to feed the contrast media to the balloon and therefore the minimum achievable diameter of the balloon catheter.

Changes to the balloon structure, for instance to incorporate into the balloon walls radiopaque or echogenic elements, can alter the characteristics and performance of the balloon and are therefore not always advantageous.

Balloon catheter assemblies suitable for imaging are disclosed, for example, in US-5,967,988, US-2005/0074406, US-6,106,473, US-2009/0318746 and US-4,349,033.

US2002/0054852 describes a method and apparatus for treating a specific site in a mammal with a drug, and for imaging the progress of the drug through the body.

US2010/0305389 discloses a device for insertion into a patient and a method for imaging the device by x-ray using barium iodide.

US4364392 describes a method and apparatus for occluding a body vessel, the apparatus comprising a balloon which is filled with solid filler particles.

### Disclosure of the Invention

The present invention seeks to provide an improved balloon catheter assembly as defined in claim 1 and subsequent dependent claims 2-11 and in particular having a balloon which is more readily visible by imaging, in particular ultrasonic or magnetic imaging.

According to an aspect of the present invention, there is provided a balloon catheter assembly for endoluminal location within a patient, the assembly including a balloon catheter having a proximal and a distal end; an impervious inflatable balloon attached to the catheter at or proximate its distal end; the catheter including at least one inflation port at its distal end for the passage of inflation fluid into and out of the balloon; and a source of inflation fluid for inflating the balloon couplable to the catheter, said inflation fluid including a plurality of radiopaque or echogenic particles therein; wherein the particles have a diameter of between 2 to 10 micrometres.

Advantageously, the particles, which may be microcapsules or pellets, are in suspension in the inflation fluid.

The particles, microcapsules or pellets, are preferably substantially spherical. In the preferred embodiment, the particles, microcapsules or pellets have a diameter smaller than the wavelength of ultrasound used.

The particles affect the passage of ultrasonic waves directed to the balloon, by radiopacity or echogenicity, thereby making the balloon visible under ultrasonic imaging. Providing the particles within the inflation fluid allows the balloon to be of a conventional form and also can avoid the need for contrast media of the type commonly used. In the preferred embodiment, the particles are suspended in saline solution does not have the viscosity of contrast media and therefore can be provided through smaller diameter channels and which can provide faster inflation and deflation of the balloon. Moreover, saline solution does not suffer from the poorer biocompatibility of at least some contrast media.

The particles, microcapsules or pellets may be at least one of: solid and hollow. There is a number of possible particles which would be suitable. Examples include collagen microspheres, iodipamide ethyl ester, which can be used in solid form. Hollow particles could be filled with a contrast media and in one embodiment are filled with a perfluorochemical, that is a perfluorocarbon. Other embodiments include hollow microcapsules or pellets filled with air, gas, or a gel or fluid of density different to that of blood. Gas filled particles or capsules can be of a heavy gas such as perfluoropropane, dodecafluoropentane, octafluoropropane, perfluorobutane, perfluorocarbon, perfluorohexane, sulphur hexafluoride, nitrogen and so on. These gases can give the particles good longevity as well as good echogenicity. Moreover, heavy gases have relatively low water solubility and thus are less likely to suffer from leakage from the particles or capsules.

It is preferred that the particles, microcapsules or pellets are provided in a concentration of 5 to 100 microlitres of particles, microcapsules or pellets per millilitre of inflation fluid. This, it has been found, provides good visibility of the balloon without being of too great a concentration in the carrier fluid.

The particles, microcapsules or pellets can made of polymethylmethacrylate (PMMA), PLA, PLGA, poly (methyl urea), amphiphilic block copolymers or polysaccharides, for example.

Advantageously, the microcapsules or pellets are of a reflective material, an opaque material and/or of a shape to promote Rayleigh scattering

There is also disclosed a method of inflating a balloon of a medical balloon catheter assembly, not forming part of the invention, which assembly includes a balloon catheter having a proximal and a distal end; an inflatable balloon attached to the catheter at or proximate its distal end; the catheter including at least one inflation port at its distal end for the passage of inflation fluid into and out of the balloon; the method including the step of:
inflating the balloon with an inflation fluid including a plurality of radiopaque or echogenic particles therein.

The particles, which may be microcapsules or pellets, can be in suspension in the inflation fluid.

The particles, microcapsules or pellets may have a diameter smaller that the wavelength of ultrasound applied during the step of inflation of the balloon.

### Brief Description of the Drawings

Embodiments of the present invention are described below, by way of example only, with reference to the accompanying drawings, in which:
Figure 1A is a schematic diagram of an embodiment of balloon catheter assembly;
Figure 2 is an enlarged side elevational view of an embodiment of balloon of the assembly of Figure 1; and
Figures 3 and 4 are, respectively, side elevational views of a part of two balloon catheter assemblies subjected to ultrasonic imaging, Figure 3 showing a conventional assembly and Figure 4 showing the embodiment of Figures 1 and 3.

### Description of the Preferred Embodiments

Described below are various embodiments of balloon catheter assembly provided with one or more echogenic elements. By echogenic it is meant elements which are visible to imaging, in the preferred embodiment to ultrasound imaging. As will be apparent from the teachings herein, the preferred embodiments provide elements which contribute or cause Rayleigh scattering of ultrasonic wave energy, although the elements could otherwise be absorbent (radiopaque) or reflective of ultrasonic waves.

The teachings herein can be used with any balloon catheters, including dilatation balloons, occlusion balloons, scoring or cutting balloons and other balloon structures used for angioplasty procedures, as well as balloons used in the deployment of implantable medical devices.

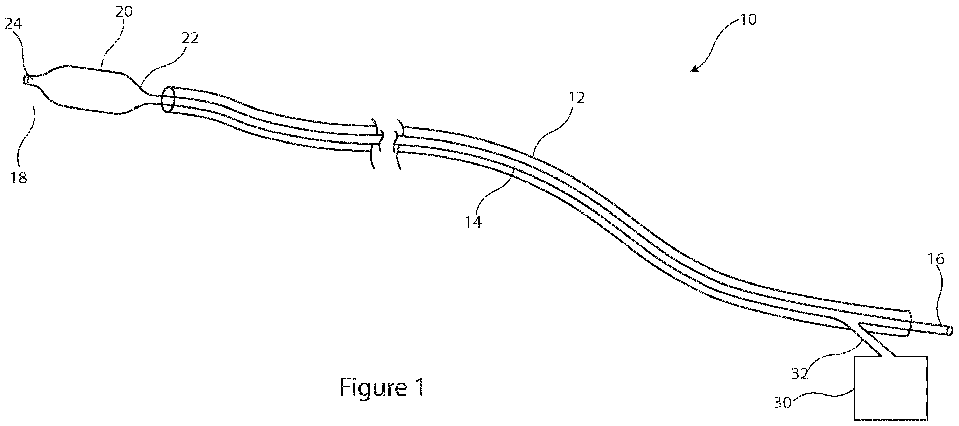

Referring to Figure 1, there is shown in schematic form an embodiment of balloon catheter assembly 10 according to the present invention. The principal components of the assembly 10 are analogous to conventional catheter assemblies and comprise an outer sheath 12 which in use covers the entirety of the balloon catheter during the endoluminal insertion of the assembly 10 into a patient. Within the sheath 12 there is provided a balloon catheter 14 which has a proximal end 16 and a distal end 18. The distal end 18 may typically be provided with a dilator tip (not shown in the drawing).

At the distal end 18, there is provided a medical balloon 20 which comprises proximal and distal ends 22, 24 fixed in fluid-tight manner to the catheter 14. The method of fixing these ends 22, 24 of the medical balloon 20 could be any of the conventional ones known in the art.

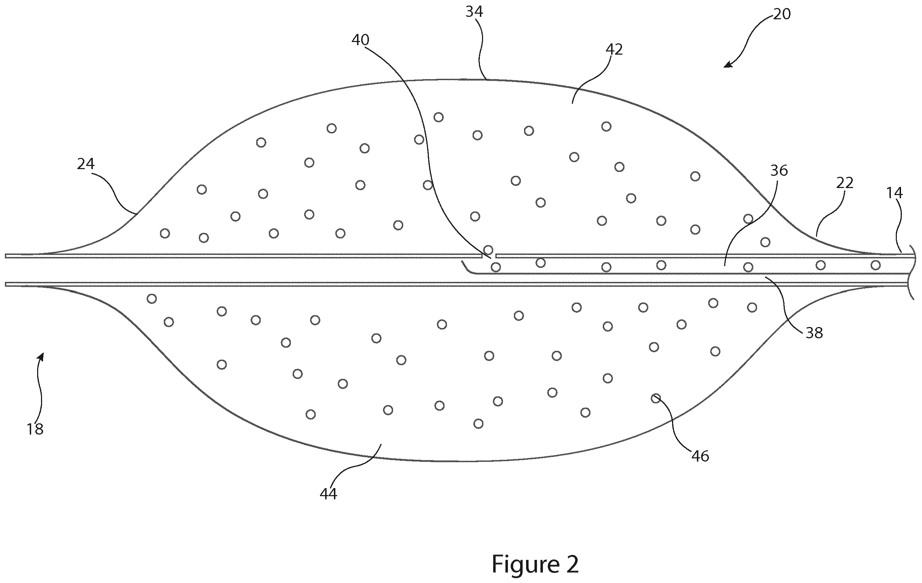

The catheter 16 includes therewithin one or more lumens, not shown in Figure 1 but described below in connection with Figure 2, one of which lumens couples to the interior of the balloon 20 for inflating and deflating the balloon. One or more other lumens are typically provided for other purposes, such as for receiving a guide wire.

The assembly 10 also includes an inflation fluid source 30 which is coupled by means of a suitable conduit to the catheter 16 and in particular to the inflation/deflation lumen within the catheter 16 used for inflating and deflating the balloon 20. The nature of the inflation fluid source 30 is described in further detail below.

The sheath 12 is able to move along the length of the catheter 14 from the retracted position as shown in Figure 1, in which the distal end and in particular the medical balloon 20 are exposed, to a covering position in which the sheath 12 covers the balloon 20 and distal end 18 of the catheter 14. In the covering position, the balloon 20 is typically in a deflated state and wrapped around the catheter 22, as is conventional in the art.

The balloon 20 can be made of any of a variety of balloon materials including, for example polyether block amide (Pebax), nylon, preferably nylon 12, polyethylene, polyurethane and any other suitable material. It may be made of a single layer of material or a plurality of layers of material and may be compliant or non-compliant.

Furthermore, the balloon 20 can be of a type used to perform any of the functions commonly associated with medical balloons, as described above. The balloon 20 can, therefore, have a conventional form of a general cylindrical body portion with generally conical end portions, but may have a variety of other shapes including hourglass and so on. The balloon 20 may also include scoring or cutting elements thereon.

Referring now to Figure 2, this illustrates in enlarged form the balloon 20 of Figure 1, with only a part of the catheter 14 being visible.

The balloon 20 has a balloon wall 34 which is substantially cylindrical with general conical end portions 22, 24 ending in necks which are sealed to the catheter-in fluid tight manner. The balloon is shown in an inflated condition in Figure 2 and thus with the wall 34 extending radially outwardly of the catheter 14.

The catheter 14 includes, in this example, first and second lumens 36, 38, which are shown in schematic form in Figure 2. The lumen 38, which extends the whole length of the catheter 14, that it from its proximal end 16 through to its distal end 18, provides a passage through the catheter 14 for a guide wire (not shown). The lumen 36, on the other hand, couples to the conduit 32, and thus to the inflation fluid source 30, and ends at a port 40 in the catheter 14. The port 40 is typically an aperture or hole in the wall of the catheter. The port 40 thus provides for fluid communication between the interior chamber 42 of the balloon 20 and the fluid of the source 30.

Figure 2 shows the balloon 20 inflated with an embodiment of inflation fluid. This inflation fluid includes a liquid 44, preferably a saline solution. In this embodiment, suspended in the liquid 44 are a plurality of particles 46 of an echogenic or radiopaque material. In some embodiments, the particles 46 may be both echogenic and radiopaque.

In the preferred embodiments, the particles 46 are provided in a sufficient concentration (typically sufficient number per unit volume of liquid 44) to provide an amount of reflection or absorption of ultrasonic waves directed to the balloon 20 to give a measurable effect and in particular to be visible under ultrasonic imaging. It has been found that particles in a concentration of around 5 to 100 microlitres of particles, microcapsules or pellets per millilitre of inflation fluid gives good visibility of the balloon 20 when subjected to ultrasonic imaging. It will be appreciated that larger particle can be less numerous in number, whereas smaller particles would typically be greater in number to provide good characteristics in terms of visibility. The person skilled in the art will be able to determine an optimum particle size for the particular medical balloon within which they are intended to be used.

The particles 46 are preferably substantially spherical or otherwise rounded (for example oval) to give improved echogenicity in in particular to promote Rayleigh scattering of ultrasonic waves. If the case of radiopaque particles 46, these may also be substantially spherical or otherwise rounded but may have other shapes.

It has been found that particles having a diameter of between 1 to 100 micrometres provides good visibility of the balloon. It is preferred that the particles have a diameter of between about 2 to about 10 micrometres, which gives good visibility and good flow characteristics through the lumen 36, allowing the latter to be of small diameter.

The particles, which most preferably are made of a biocompatible material, may be microcapsules or pellets and may be solid or hollow. In the case of hollow microcapsules or pellets, these may be filled with air, gas, a gel or a fluid. There is a number of possible particles which would be suitable. Examples include collagen microspheres, iodipamide ethyl ester, which can be used in solid form. Hollow particles could be filled with a contrast media and in one embodiment are filled with a perfluorochemical, that is a perfluorocarbon. Other embodiments include hollow microcapsules or pellets filled with air, gas, or a gel or fluid of density different to that of blood. Gas filled particles or capsules can be of a heavy gas such as perfluoropropane, dodecafluoropentane, octafluoropropane, perfluorobutane, perfluorocarbon, perfluorohexane, sulphur hexafluoride, nitrogen and so on. These gases can give the particles good longevity as well as good echogenicity. Moreover, heavy gases have relatively low water solubility and thus are less likely to suffer from leakage from the particles or capsules.

It has been found that particles made of polymethylmethacrylate (PMMA) perform well, that is have good echogenicity in use. Other examples include poly(lactic acid) (PLA), poly(lactic-co-glycolic acid) (PLGA), PCL, poly(methyl urea), amphiphilic block copolymers or polysaccharides.

The particles 46 may equally be made of any other ultrasonically reflective or opaque material.

Although saline solution is the preferred fluid 44, other embodiments may use a fluid which includes a contrast medium. In such embodiments, the fluid 44 may include only a relative weak concentration of contrast medium, thereby to prevent or minimise the problems of relatively high viscosity and toxicity of at least some contrast media known in the art.

It will be appreciated that the port 40 in the catheter 14 as well as the lumen 36, will be of a dimension to be able easily to allow the passage of the particles 46 therethrough when in the fluid 44.

Referring again to Figure 1, the source 30 of inflation fluid includes a liquid 44 and particles 46 in suspension and a mechanism (of conventional form) for pumping the inflation fluid into the balloon 20 through the lumen 36 as well as for withdrawing fluid from the balloon 20 when it is desired to deflate and collapse the balloon 20, typically after conclusion of the medical procedure. The pumping mechanism could be a syringe, a drop bag or a dedicated pump.

The source 30 can be filled with liquid 44 in which the particles 46 are already in suspension. The source 30 may equally be provided with a mechanism, such as a mixing or shaking device, to ensure that the particles 46 brought into and remain in suspension. In another embodiment, the source 30 may include a separate feed or hopper of particles 46 which are mixed into the liquid 44 at the time of pumping of the fluid into the catheter 14 and therefrom into the balloon 20. Of relevance is that the particles 46 are carried by the liquid 44 into the balloon 20.

It is not necessary for the particles 46 to remain suspended in the liquid 44 once injected into the balloon 20, and it is not excluded that in some embodiments the particles 46 may be of a material which causes them to attach themselves to the inner wall 34 of the balloon 20 once they have been injected into the chamber 42.

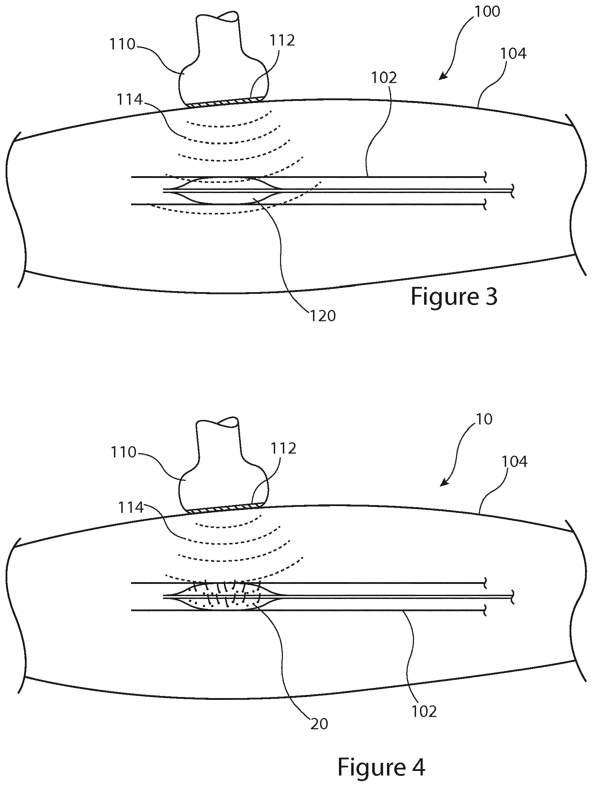

Referring now to Figures 3 and 4, these show, respectively, a conventional balloon assembly 100 and a balloon assembly 10 of the type described and taught herein.

In Figure 3, the balloon catheter 120 is shown located within a vessel 102 of a patient. In this illustrative example, the vessel 102 is within a part of the patient's body, for instance an arm 104 or other limb. The balloon catheter 120 is of conventional type and filled with saline solution or other equivalent liquid. The balloon catheter 120 is not filled with contrast media of the type known in the art.

An ultrasonic probe 110 is placed in abutment with the body part 104, just above the vessel 102, and coupled with a suitable coupling medium 112, such as a gel, as known in the art. Ultrasonic waves 114 emanating from the probe 110 pass through the body part 104 and the vessel 102. The balloon catheter 120 does not substantially alter the ultrasonic waves 114, which thus pass through the balloon catheter 120 with virtually no absorption or reflection of the waves 114. As a result, the balloon catheter 120 appears substantially invisible under imaging.

The convention in the art is to fill the balloon 120 with a contrast media. However, as explained above, contrast media is generally viscous when in the concentration required to provide adequate radiopacity and also has toxicity, leading to risks to the patient should the balloon 120 burst in use.

Referring now to Figure 4, there is shown the embodiment of balloon catheter assembly 10 of Figures 1 and 2 within the lumen 102 of the body part 104 shown in Figure 3. The ultrasonic probe 110, with coupling gel 112, is located in the same position as in Figure 3 and operated in the same manner to generate the ultrasonic waves 114.

In contrast to the example of Figure 3, the particles 46 within the balloon 20, which in this example are echogenic, promote Rayleigh scattering. As a result, the particles 46 reflect a part of the ultrasonic energy generated by the probe 110 back towards the probe, as shown in Figure 4. This reflection can be detected by the probe 110 back towards the probe, as shown in Figure 4. This reflection can be detected by the probe 110, in the form of an image having the shape of the balloon 20. Thus, the presence of particles 46 within the inflation fluid in the balloon chamber 42 makes the balloon 20 visible under imaging. Embodiments which use radiopaque particles 46 will be equally visible under ultrasonic imaging.

These embodiments are able to use an inflation liquid 44 which does not suffer from the disadvantages of known contrast media and in particular can use an inflation liquid 44 which is of relatively low viscosity and low toxicity, such as saline solution.

The embodiments described herein can provide visibility to a medical balloon 20 which would otherwise be substantially invisible under ultrasonic imaging and yet in a manner which does not suffer from the disadvantages of conventional arrangements.

As described above, the balloon 20 could be filled with a liquid 44 which also includes some contrast media to enhance the visibility of the balloon 20. In this embodiment, the contrast media can be of low viscosity and/or low concentration in light of the presence of the particles 46 within the inflation fluid.

It is to be appreciated that the balloon is impervious. It is to be understood that only some embodiments are described above which would be apparent to the skilled person having regard to the teachings herein and that the described embodiments are not intended to be limiting of these teachings.

## Claims

1. A balloon catheter assembly (10) for endoluminal location within a patient, the assembly including a balloon catheter (14) having a proximal (16) and a distal (18) end; an impervious inflatable balloon (20) attached to the catheter at or proximate its distal end; the catheter including at least one inflation port (40) at its distal end for the passage of inflation fluid into and out of the balloon; and a source (30) of inflation fluid (44) for inflating the balloon couplable to the catheter (14), said inflation fluid (44) including a plurality of radiopaque or echogenic particles (46) therein; wherein the particles have a diameter of between 2 to 10 micrometres.

2. A balloon catheter assembly (10) according to claim 1, wherein the particles (46) are in suspension in the inflation fluid (44).

3. A balloon catheter assembly (10) according to any preceding claim, wherein the particles (46) are substantially spherical.

4. A balloon catheter assembly (10) according to any preceding claim, wherein the particles (46) are microcapsules or pellets.

5. A balloon catheter assembly (10) according to claim 4, wherein the microcapsules or pellets are at least one of: solid and hollow.

6. A balloon catheter assembly (10) according to claim 5, wherein hollow microcapsules or pellets are filled with air, gas or a fluid.

7. A balloon catheter assembly (10) according to any preceding claim, wherein the particles (46) are provided in a concentration of 5 to 100 microlitres per millilitre of inflation fluid (44).

8. A balloon catheter assembly (10) according to any preceding claim, wherein the particles (46) are made of a biocompatible material.

9. A balloon catheter assembly (10) according to claim 8, wherein the particles are made of polymethylmethacrylate (PMMA), poly(lactic acid) (PLA), poly(lactic-co-glycolic acid) (PLGA), poly (methyl urea), amphiphilic block copolymers or polysaccharides.

10. A balloon catheter assembly (10) according to any preceding claim, wherein the particles (46) are of at least one of: a reflective material, an opaque material and of a form to promote Rayleigh scattering.

11. A balloon catheter assembly (10) according to any preceding claim, wherein the inflation fluid (44) is at least of: saline solution; and inflation fluid including contrast medium.

## Patentansprüche

1. Ballonkatheteranordnung (10) zur endoluminalen Platzierung in einem Patienten, wobei die Anordnung einen Ballonkatheter (14) mit einem proximalen Ende (16) und einem distalen Ende (18) aufweist; einen undurchlässigen aufblasbaren Ballon (20), der am Katheter an seinem oder in der Nähe seines distalen Endes befestigt ist; wobei der Katheter mindestens eine Aufblasöffnung (40) an seinem distalen Ende zum Durchleiten von Aufblasfluid in den und aus dem Ballon aufweist; und eine Quelle (30) eines Aufblasfluids (44) zum Aufblasen des Ballons, die mit dem Katheter (14) verbindbar ist, wobei das Aufblasfluid (44) eine Vielzahl von röntgenopaken oder echogenen Partikeln (46) darin aufweist; wobei die Partikel einen Durchmesser zwischen 2 und 10 Mikrometern aufweisen.

2. Ballonkatheteranordnung (10) nach Anspruch 1, wobei die Partikel (46) in dem Aufblasfluid (44) suspendiert sind.

3. Ballonkatheteranordnung (10) nach einem der vorhergehenden Ansprüche, wobei die Partikel (46) im Wesentlichen kugelförmig sind.

4. Ballonkatheteranordnung (10) nach einem der vorhergehenden Ansprüche, wobei die Partikel (46) Mikrokapseln oder Pellets sind.

5. Ballonkatheteranordnung (10) nach Anspruch 4, wobei die Mikrokapseln oder Pellets zumindest eines der folgenden sind: massiv und hohl.

6. Ballonkatheteranordnung (10) nach Anspruch 5, wobei hohle Mikrokapseln oder Pellets mit Luft, Gas oder einem Fluid gefüllt sind.

7. Ballonkatheteranordnung (10) nach einem der vorhergehenden Ansprüche, wobei die Partikel (46) in einer Konzentration von 5 bis 100 Mikroliter pro Milliliter Aufblasfluid (44) bereitgestellt werden.

8. Ballonkatheteranordnung (10) nach einem der vorhergehenden Ansprüche, wobei die Partikel (46) aus einem biokompatiblen Material bestehen.

9. Ballonkatheteranordnung (10) nach Anspruch 8, wobei die Partikel aus Polymethylmethacrylat (PMMA), Poly(milchsäure) (PLA), Polylactid-co-Glycolid (PLGA), Poly(methylharnstoff), amphiphilen Blockcopolymeren oder Polysacchariden bestehen.

10. Ballonkatheteranordnung (10) nach einem der vorhergehenden Ansprüche, wobei die Partikel (46) zumindest eines der folgenden sind: ein reflektierendes Material, ein opakes Material und eine Form zur Förderung von Rayleigh-Streuung.

11. Ballonkatheteranordnung (10) nach einem der vorhergehenden Ansprüche, wobei das Aufblasfluid (44) eines der folgenden ist: Kochsalzlösung; und ein Inflationsfluid, das Kontrastmittel enthält.

## Revendications

1. Ensemble (10) cathéter à ballonnet à placer dans une lumière du corps d'un patient, l'ensemble comprenant un cathéter (14) à ballonnet comportant une extrémité proximale (16) et une extrémité distale (18) ; un ballonnet gonflable (20) imperméable fixé au cathéter au niveau ou à proximité de son extrémité distale ; le cathéter comprenant au moins un orifice de gonflage (40) à son extrémité distale pour permettre à un fluide de gonflage d'entrer dans le ballonnet et d'en sortir ; et une source (30) de fluide de gonflage (44) pour gonfler le ballonnet, pouvant être accouplée au cathéter (14), ledit fluide de gonflage (44) contenant une pluralité de particules (46) radio-opaques ou échogènes,
dans lequel les particules ont un diamètre compris entre 2 et 10 microns.

2. Ensemble (10) cathéter à ballonnet selon la revendication 1, dans lequel les particules (46) sont en suspension dans le fluide de gonflage (44).

3. Ensemble (10) cathéter à ballonnet selon l'une quelconque des revendications précédentes, dans lequel les particules (46) sont sensiblement sphériques.

4. Ensemble (10) cathéter à ballonnet selon l'une quelconque des revendications précédentes, dans lequel les particules (46) sont des microcapsules ou des pastilles.

5. Ensemble (10) cathéter à ballonnet selon la revendication 4, dans lequel les microcapsules ou pastilles sont pleines et/ou creuses.

6. Ensemble (10) cathéter à ballonnet selon la revendication 5, dans lequel les microcapsules ou pastilles creuses sont remplies d'air, de gaz ou d'un fluide.

7. Ensemble (10) cathéter à ballonnet selon l'une quelconque des revendications précédentes, dans lequel les particules (46) sont utilisées à une concentration de 5 à 100 microlitres par millilitre de fluide de gonflage (44).

8. Ensemble (10) cathéter à ballonnet selon l'une quelconque des revendications précédentes, dans lequel les particules (46) sont faites d'une matière biocompatible.

9. Ensemble (10) cathéter à ballonnet selon la revendication 8, dans lequel les particules sont faites de poly(méthacrylate de méthyle) (PMMA), d'acide polylactique (PLA), d'acide poly(lactique-coglycolique) (PLGA), de poly(méthylurée), de copolymères à blocs amphiphiles ou de polysaccharides.

10. Ensemble (10) cathéter à ballonnet selon l'une quelconque des revendications précédentes, dans lequel les particules (46) sont constituées d'une matière réfléchissante et/ou d'une matière opaque et/ou ont une forme favorisant la diffusion de Rayleigh.

11. Ensemble (10) cathéter à ballonnet selon l'une quelconque des revendications précédentes, dans lequel le fluide de gonflage (44) est au moins une solution saline et le fluide de gonflage comprend un produit de contraste.
